(19) Europäisches Patentamt
European Patent Office
Office européen des brevets

(11) Publication number: 0 322 983 A1

# EUROPEAN PATENT APPLICATION

(21) Application number: 88203021.6

(22) Date of filing: 27.12.88

(51) Int. Cl.4: C07C 47/575 , C07C 49/84 , C07C 43/29 , C07C 69/78 , C07C 45/63 , C07C 67/42 , C07C 37/055

(30) Priority: 28.12.87 US 138279
18.08.88 US 233385

(43) Date of publication of application: 05.07.89 Bulletin 89/27

(84) Designated Contracting States: AT BE CH DE ES FR GB IT LI NL

(71) Applicant: THE DOW CHEMICAL COMPANY
2030 Dow Center Abbott Road P.O. Box 1967
Midland Michigan 48640-1967(US)

(72) Inventor: Orvik, Jon A.
1164 Lincoln Avenue No. 338
Walnut Creek California 94596(US)
Inventor: Pearson, Norman R.
3049 Naranja Drive
Walnut Creek California 94598(US)
Inventor: Haag, Anthony P.
1163 E. Stewart
Midland Michigan 48640(US)
Inventor: Adaway, Timothy J.
3117 Lambros
Midland Michigan 48640(US)
Inventor: Halpern Mark E.
2905 Blairmont Drive
Midland Michigan 48640(US)
Inventor: Wujek Dennis G.
5013 Stony Creek Drive
Midland Michigan 48640(US)

(74) Representative: Smulders, Theodorus A.H.J., Ir. et al
Vereenigde Octrooibureaux Nieuwe Parklaan 107
NL-2587 BP 's-Gravenhage(NL)

## (54) Phenoxyphenoxypropionates, intermediates thereof and methods of preparation.

(57) A process for preparing substituted phenoxyphenols which are useful in the preparation of herbicidal (phenoxyphenoxy)propionates is disclosed. The process involves the oxidation of substituted phenoxyphenones to the corresponding phenoxyphenyl esters and their conversion to the desired phenoxyphenol. Novel intermediates for the process are similarly disclosed.

EP 0 322 983 A1

## PHENOXYPHENOXYPROPIONATES, INTERMEDIATES THEREOF AND METHODS OF PREPARATION

The present invention concerns process for preparing (phenoxyphenoxy)propionate compounds, and certain novel intermediates thereof.

Various (phenoxyphenoxy)propionate compounds are known to possess herbicidal and plant-growth regulating properties. For example, see U.S. Patents 3,954,442; 4,550,192; 4,332,961; 4,332,960; 4,370,489; 4,276,080 and British Patent Specification 1,577,181. Also, processes for preparing enantiomers are described in U.S. Patents 4,531,969 and 4,532,328. In U.S. 4,252,980 a process is taught for preparing the methyl ester.

Processes for making selected intermediates used to prepare (phenoxyphenoxy)propionates are taught in U.S. Patents 3,721,703; 4,238,626; 4,252,980; 4,301,295; 4,309,547 and 4,391,995. Although these processes are effective, a number of them utilize diazonium reactions which are potentially explosive when employed on an industrial scale. It is desirable to find methods for preparing phenoxyphenoxy compounds which are as safe or safer than those previously taught. It is also desirable to find a method which employs a fewer number of steps than other known processes. It is also desirable to find methods where yields are as good or as better than previously known processes.

The present invention is directed to the preparation of (phenoxyphenoxy)propionate compounds and to novel intermediates useful in their preparation.

More specifically, the present invention is directed to the preparation of and to novel phenoxyphenones of the formula:

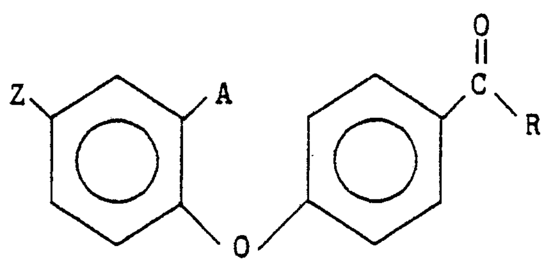

(I)

wherein:

Z is halogen or hydrogen (H);

A is halogen or H; and

R is H, $C_1$-$C_{10}$ alkyl, phenyl or substituted phenyl of the formula

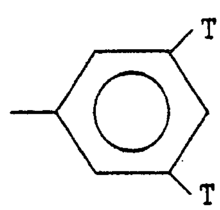

wherein T is independently halogen or H.

In a more limited embodiment, the present invention is directed to specific (4-bromo-phenoxy)phenones and processes for their preparation via bromination procedures.

The present invention is also directed to the preparation of and to novel phenoxyphenyl ester compounds of the formula:

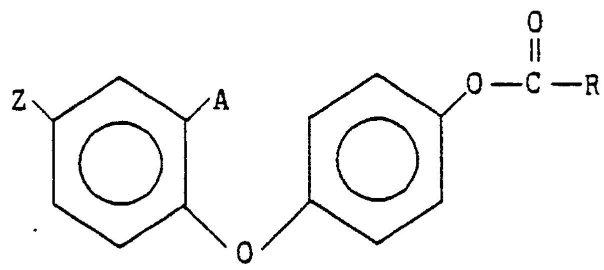

(II)

wherein:
Z is halogen or hydrogen (H);
A is halogen or H; and
R is H, $C_1$-$C_{10}$ alkyl, phenyl or substituted phenyl of the formula

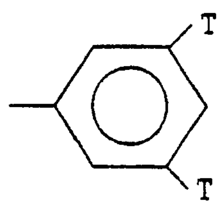

wherein T is independently halogen or H.

As used herein, "halogen" refers to fluorine, chlorine, bromine and iodine.

Such phenoxyphenones and phenoxyphenyl esters are valuable intermediates in the preparation of (phenoxyphenoxy)propionate compounds useful as herbicides and plant growth regulators.

The present invention has the advantage of providing a process for preparing (phenoxyphenoxy)-propionate compounds in yields as good as or better than other known processes. The present invention also has the advantage of providing a process which is as safe or safer than other processes previously taught. Another advantage is that it provides a process for preparing (phenoxyphenoxy)propionates in purity as good as or better than other known processes. Yet another advantage is that it provides a process which reduces the number of steps needed to prepare (phenoxyphenoxy)propionate compounds compared with other known processes. Another advantage is that novel and useful intermediates for use in such processes are provided. The same advantages apply to the optically active R-enantiomers.

The phenoxyphenols of Formula (III)

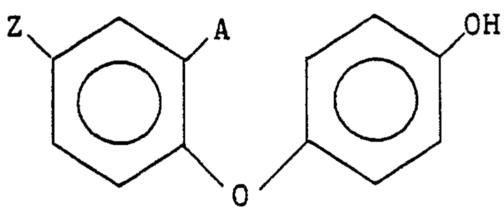

(III)

wherein:
Z is halogen or H; and
A is halogen or H,

are key intermediates in the preparation of (phenoxyphenoxy)propionate herbicides. The present invention is directed to a novel process for the preparation of phenoxyphenols of Formula (III) from phenoxyphenones of Formula (I). The process is illustrated in the following steps (*a*) and (*b*). Under certain situations, the oxidation [(step (*a*)] and the acid hydrolysis [(step (*b*)] can effectively be conducted as one step, *in situ*.

Step (a): Oxidation

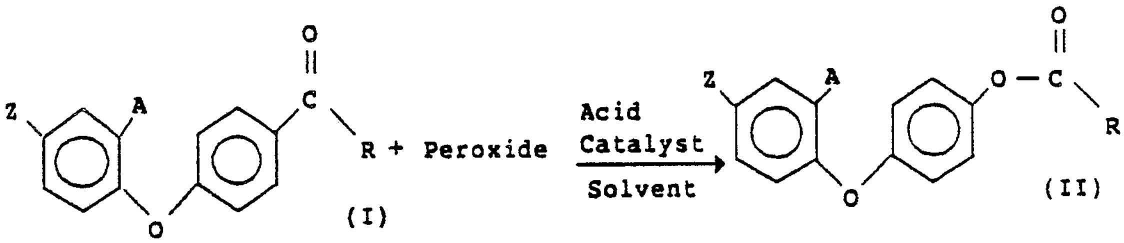


Step (b): Hydrolysis/Alcoholysis

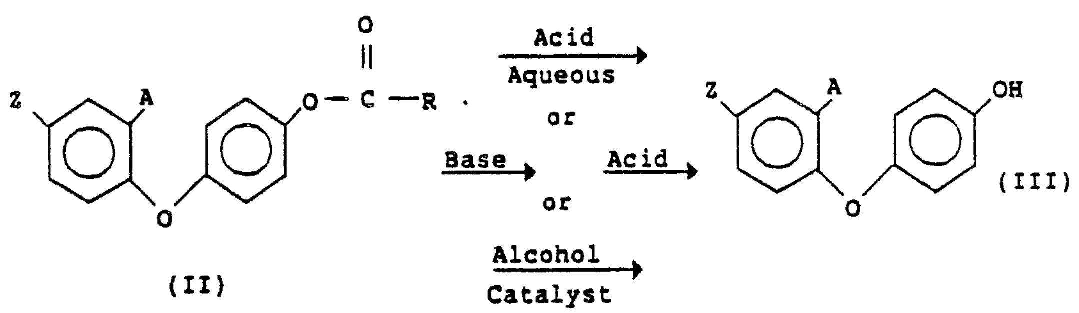


The phenoxyphenone starting materials of Formula (I) can be prepared by a variety of procedures. For example, a substituted phenol can be contacted with a phenylcarbonyl compound in the presence of a base and solvent under conditions effective to give the desired phenoxyphenone of Formula (I). The reaction is exemplified as follows:

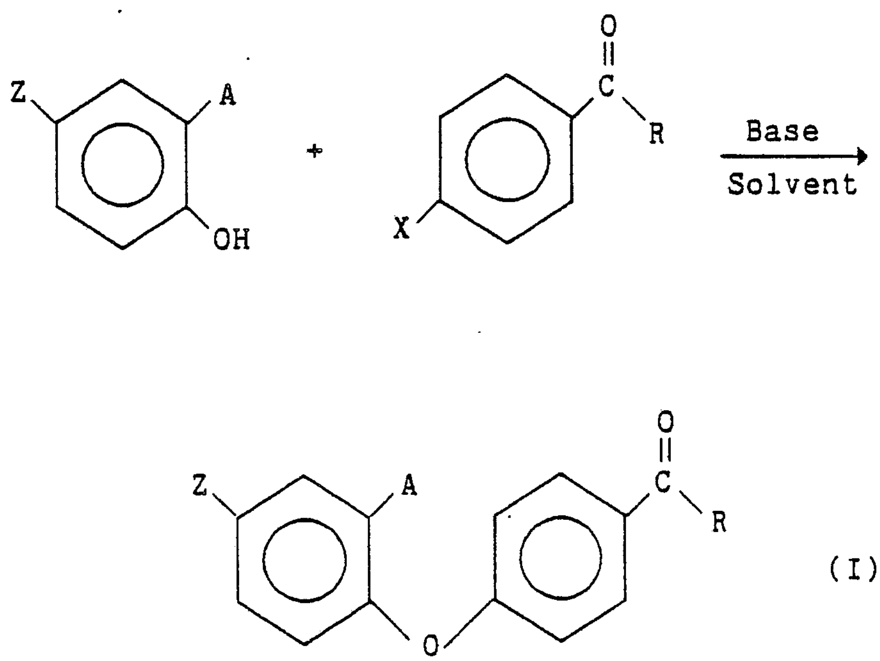


wherein:

Z is halogen or H;
A is halogen or H;
X is halogen; and
R is H, $C_1$-$C_{10}$ alkyl, phenyl or substituted phenyl of the formula

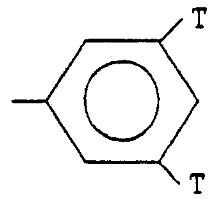

wherein T is independently halogen or H.

With respect to the phenol, Z is preferably halogen or hydrogen, more preferably bromo or H, and A is preferably halogen, more preferably fluoro. With respect to the phenylcarbonyl compound X is halogen or other appropriate leaving group, preferably fluoro or chloro; and R is $C_1$-$C_{10}$ alkyl or phenyl.

The phenol can be contacted with the phenylcarbonyl compound in molar ratios ranging from stoichiometric (i.e., 1:1) to a slight excess (i.e., 2-3 percent) of either reactant.

Suitable bases for contacting the phenol and the phenylcarbonyl compound include the bases of alkali and alkaline earth metals such as sodium hydroxide (NaOH), potassium hydroxide (KOH), sodium carbonate ($Na_2CO_3$), potassium carbonate ($K_2CO_3$) and the hydroxides and carbonates of magnesium or lithium. Other bases also include sodium or potassium methoxide.

The molar ratio of base to phenol can be in the range from about 1:1 to an excess of about 10:1 (base:phenol), preferably in amounts of slight excess over stoichiometric, e.g., 1.1-1.5:1.

Suitable inert organic solvents which can be employed include polar aprotic solvents such as N-methylpyrrolidone (NMP), dimethylsulfoxide (DMSO), hexamethylphosphoramide (HMPA), sulfolane, acetonitrile or mixtures thereof.

The reactants can be contacted at temperatures ranging from ambient to 250 degrees Centigrade (°C), preferably from 50 to 190°C. The reactants are contacted for a time sufficient to ensure substantial completion of the reaction, ranging from 15 minutes (min) to 24 hours (hr) or more. The contacting is carried out at ambient pressures, although pressures greater than ambient can be employed.

The contacting of the reactants can be carried out in the presence of air, although in order to reduce by-product formation, the contacting is preferably carried out under an inert atmosphere such as that provided by nitrogen, helium or argon, preferably nitrogen.

Antioxidants can be employed in the reaction mixture to reduce by-product formation. Such antioxidants include *t*-butylcatechol, benzothiazine, butylated hydroxytoluene (BHT), butylated hydroxyanisole (BHA) or mixtures thereof. The amount of antioxidant employed in the reaction mixture can range from 0.1 to 10 percent (%) by weight of either the phenol (I) or phenyl carbonyl reactant preferably 2 percent.

The requisite phenols can be prepared, for example, as described in U.S. Patent 4,550,192. Appropriate phenyl carbonyl compounds can be prepared, for example, by the processes taught by N. Buu-Hoi and P. Jacquignon in *Rec.Trav.Chim.* 68, 781-8 (1949) or analogous procedures thereof.

Alternatively, when R is $C_1$-$C_{10}$ alkyl, phenyl or substituted phenyl of the formula

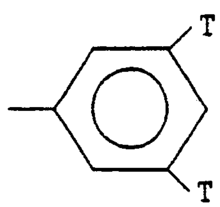

wherein T is independently halogen or H,

The phenoxyphenones of Formula (I) may be prepared by the conventional Friedel-Crafts acylation of the appropriate diphenyl ether with an acyl halide in the presence of a Lewis acid catalyst according to the following reaction:

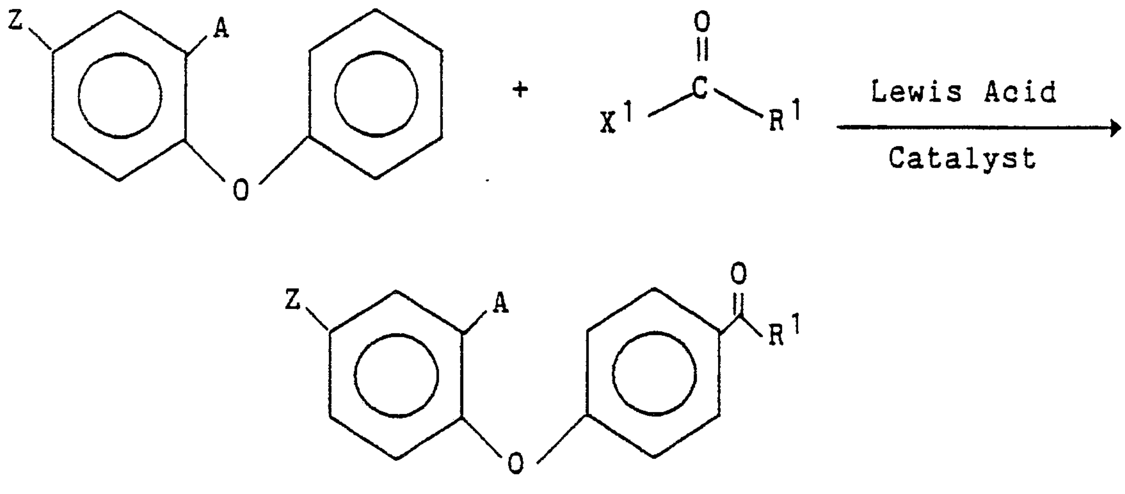


wherein:

Z is halogen or H;

A is halogen or H;

$X^1$ is halogen; and

$R^1$ is $C_1$-$C_{10}$ alkyl, phenyl or substituted phenyl of the formula

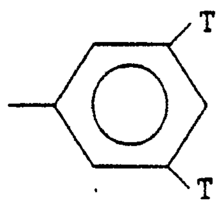

wherein T is independently halogen or H.

With respect to the diphenyl ether, Z is preferably halogen or H, more preferably bromo or H, and A is preferably halogen, more preferably fluoro. With respect to the acyl halide, $X^1$ is preferably chloro or bromo and $R^1$ is preferably substituted phenyl of the formula

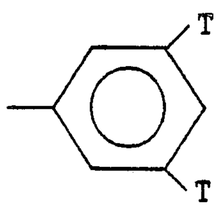

wherein T is independently halogen or H.

The diphenyl ether can be contacted with the acyl halide in molar ratios ranging from stoichiometric (i.e., 1:1) to a slight excess (i.e., 2-3 percent) of either reactant.

Suitable Lewis acid catalysts include the chlorides of aluminum, iron, tin, antimony and boron. A little more than one mole of catalyst per mole of acyl halide is required since the first mole of catalyst coordinates with the oxygen of the acylating reagent.

Suitable inert solvents which can be employed include polar solvents such as nitrobenzene and nonpolar solvents such as methylene chloride, chloroform and carbon tetrachloride.

The reactants can be contacted at temperatures ranging from 0 to 200 °C, preferably from 0 to 100 °C. The reactants are contacted for a sufficient time to ensure substantial completion of the reaction, ranging from 15 min to 24 hr or more. The contacting is carried out at ambient pressure, although pressures greater than ambient can be employed.

The requisite diphenyl ether can be prepared, for example, by the Ullmann diaryl ether synthesis. Appropriate acyl halides can be prepared by the reaction between the corresponding acid and an inorganic

acid halide such as, for example, thionyl chloride.

The desired phenoxyphenone (I), prepared from either the phenol and phenylcarbonyl or the diphenyl ether and acyl halide starting materials can be recovered by conventional procedures such as distillation, recrystallization, phase separation or extraction from water into a water-immiscible organic solvent such as methylene chloride, perchloroethylene, carbon tetrachloride ($CCl_4$) or chloroform ($CHCl_3$). The phenoxyphenone can be further purified by repeating the above procedures or can be tised in its unpurified form for subsequent reactions.

By following the preparative procedures as set forth in the examples herein and by employing the appropriate starting materials or reactants, the following compounds are prepared, as set forth in Table 1.

Table 1

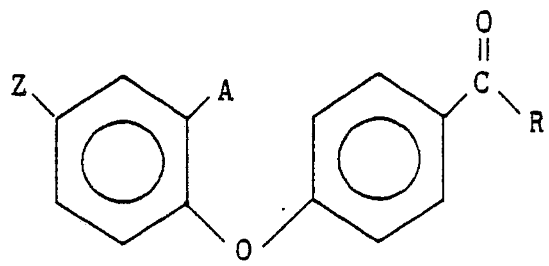

| Z | A | R |
|---|---|---|
| H | H | Φ (phenyl) |
| H | F | Φ |
| H | F | $CH_3$ |
| H | F | H |
| H | F | 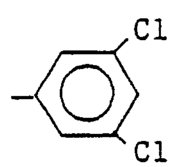 |
| Br | F | Φ |
| Br | F | $CH_3$ |
| Br | F | H |
| H | Cl | Φ |
| H | Cl | $CH_3$ |
| H | Cl | H |
| Cl | Cl | Φ |
| Cl | Cl | $CH_3$ |
| Cl | Cl | H |
| Br | Cl | Φ |
| Br | Cl | $CH_3$ |
| Br | Cl | H |

In the particular situation wherein as to the phenoxyphenone of Formula I, Z = H, the hydrogen can be converted to bromo before proceeding with further oxidation in order to prepare (4-bromophenoxyphenoxy)-propionate compounds. The 4-brominated phenoxyphenone can be prepared by contacting a 4-hydrogen phenoxyphenone of Formula (I′) with bromine in the presence of a metal halide catalyst and solvent to yield the 4-bromophenoxyphenone of Formula (I″). The reaction is exemplified as follows:

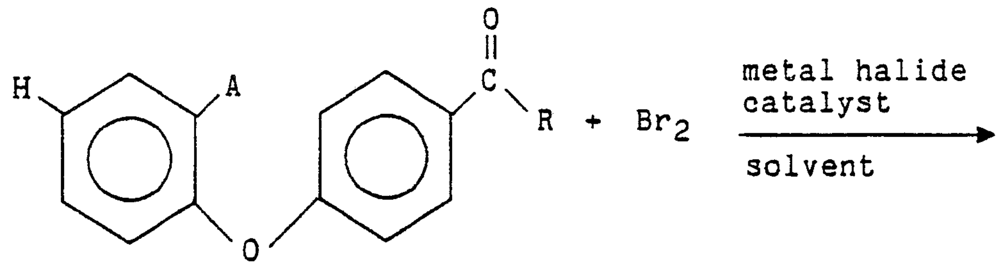


(I')

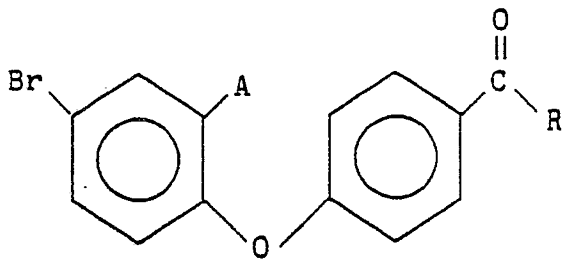

(I")

wherein:

A and R are as defined hereinbefore.

Bromine ($Br_2$) is a non-metallic halogen element which has the property of being a dark, reddish-brown liquid.

The metal halide catalyst is of the formula

$M^nX_n$

wherein:

M is aluminum (Al), titanium (Ti), iron (Fe) or boron (B);

X is chloro, bromo or fluoro; and

n is an integer which is the oxidation state of the metal.

Preferably M is aluminum; also preferred is that X is chloro or bromo and that n is 3. For titanium, preferably n is 4. Catalysts which can conveniently be employed include aluminum chloride, aluminum bromide, titanium chloride, titanium bromide, iron chloride, iron bromide and the like; aluminum chloride or bromide are usually preferred.

The metal halide catalyst is used in amounts slightly greater or much greater than the molar equivalents of 4-H-phenoxyphenone (I′) employed. A molar equivalence is defined as one mole of catalyst per mole of 4-H-phenoxyphenone (I′). The amounts of metal halide catalyst can range from 10 to 1.001 moles metal halide catalyst to 1 mole 4-H-phenoxyphenone (I′), more preferably from 1.5-1.1:1 (moles metal halide catalyst:moles 4-H-phenoxyphenone (I′)). The metal halide catalyst should be maintained anhydrous or as water-free as possible, since water can chemically react and deactivate the catalyst.

Contacting of the ingredients is performed in the presence of a solvent resistant or inert to bromination. Such inert solvents include, but are not limited to, chlorinated aliphatic hydrocarbons such as methylene chloride, carbon tetrachloride, chloroform, perchloroethylene, 1,2-dichloroethane, trichloroethylene and the like. The solvent employed can range from an amount sufficient to at least slurry the ingredients up to any amount which would homogenize the ingredients. Typically, the concentration of the phenoxyphenone (I′) is from 5 to 50 percent by weight of a solution containing an inert solvent; preferably from 10 to 20 percent by weight.

The temperatures, pressures and times employed should be sufficient to yield the desired 4-bromophenoxyphenone of Formula (I″). The temperature can range from -30 °C to the boiling point of bromine, preferably at ambient temperatures or lower due to the ease of handling the bromine and/or

can be employed to dissolve any starting materials in solid or crystalline form. Solvents which can be employed include the $C_1$-$C_6$ alcohols, such as methanol, ethanol, butanol, hexanol, and the like. Other solvents include acetonitrile.

Acetic acid which can serve as both a reactant and as a solvent, can be employed in excess amounts relative to the phenoxyphenone (I).

The process of Step (*a*) is conducted at temperatures, pressures, and times effective to convert the phenoxyphenone of Formula (I) to the phenoxyphenyl ester of Formula (II). Such temperatures can range from 0° C to the temperatures which would cause significant decomposition of the peroxide, preferably from 0 to 80° C, more preferably from 0 to 60° C, most preferably from 20 to 50° C. The process of Step (*a*) can be conducted at pressures greater than ambient, although ambient pressures are preferred.

The ingredients in Step (*a*) can be contacted in any particular order. Preferably, though, the phenoxyphenone, solvent and acid catalyst of Step (*a*) are first contacted together followed by subsequent additions of small amounts of peroxide, due to the highly exothermic reaction of the peroxide in the reaction medium.

The desired phenoxyphenyl ester of Formula (II) can be recovered by conventional techniques such as extraction and/or distillation. For example, the reaction medium can be diluted with water and the organic phase containing the desired phenoxyphenyl ester (II) can be separated from the aqueous phase. Further purification can be accomplished by subsequent distillation, crystallization or the like.

Step (*b*): Hydrolysis/Alcoholysis

The phenoxyphenyl ester of Formula (II) can be converted to the desired phenoxyphenol of Formula (III) by three alternative methods.

Alternative 1 - *In-Situ* Acid Hydrolysis

In the *in-situ* acid hydrolysis, the phenoxyphenone (I) is converted directly to the phenoxyphenol (III). The phenoxyphenyl ester (II) is hydrolyzed in the aqueous acidic reaction media without isolation according to the following scheme:

improved selectivity of bromination. The pressure can range from ambient to pressures greater than ambient, preferably ambient pressures. The times for contacting of the ingredients can vary ranging from 15 min to 4 to 5 hr or more. The ingredients can be contacted in any order or by any convenient means. Preferably, the 4-H-phenoxyphenone (I'), solvent and catalyst are first contacted together, followed by subsequent contacting with slow and/or continuous additions of bromine due to the evolution of HBr.

The desired 4-bromophenoxyphenone (I'') can be recovered by conventional procedures such as extraction, distillation, recrystallization, filtration and the like. Typically, after completion of the reaction, the reaction mixture is diluted with water followed by separation of the organic phase containing the desired 4-phenoxyphenone (I'') followed by conventional procedures described hereinbefore.

Step (*a*): Oxidation

In Step (*a*) of the process overall, the phenoxyphenone of Formula (I) is contacted with a peroxide in the presence of an acid catalyst to give the desired phenoxyphenyl ester of Formula (II). The reaction is exemplified as follows:

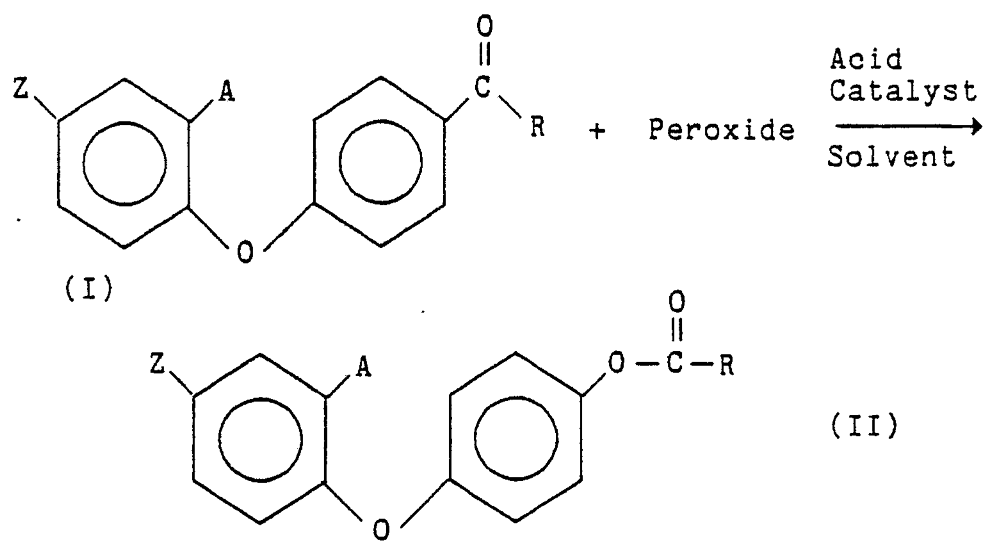


(I) (II)

wherein Z, A, and R are as defined hereinbefore.

Peroxides which can be employed include hydrogen peroxide (HOOH) alone or peracetic acid ($CH_3COOOH$) alone; the peroxide can also be mixtures of hydrogen peroxide with either acetic acid ($CH_3COOH$) or peracetic acid. Where a mixture of hydrogen peroxide and acetic acid is employed, the acetic acid is employed to regenerate additional peracetic acid for reaction with the phenoxyphenone of Formula (I). The peroxide is employed in amounts ranging from 1 to 10, preferably from 1.1 to 2.0 moles peroxide to 1 mole phenoxyphenone (I).

Where a mixture of hydrogen peroxide and acetic acid is employed, the ratio of acetic acid to peroxide can vary, ranging from 100 or more moles acetic acid per mole of peroxide to 1 mole acetic acid per mole of peroxide; preferably ratios from 15:1 (moles acetic acid:moles peroxide) to 2:1 are employed.

In the situation where peroxide is contacted with the phenoxyphenone (I) without acetic acid in amounts sufficient to oxidize the phenoxyphenone (I) to the desired phenoxyphenyl ester (II), such amounts can range from 10 to 1.1 moles peroxide per mole phenoxyphenone (I). A slight stoichiometric excess of peroxide in the range of 1.2-2:1 (moles peroxide:moles phenoxyphenone) is preferred.

The acid catalyst in the process of Step (*a*) can be any acidic catalyst which will catalyze the oxidation of the phenoxyphenone of Formula (I) by the peroxide to yield the phenoxyphenyl ester of Formula (II). Such acid catalysts include mineral acids such as sulfuric acid ($H_2SO_4$), phosphoric acid ($H_3PO_4$) or organic acids such as acetic acid or acidic ion exchange resins such as Dowex® MSC-1-H ion exchange resin (trademark of The Dow Chemical Company).

The acid catalyst is employed in an amount ranging from 0.1 to 100 moles acid catalyst per mole phenoxyphenone (I), preferably 1-3:1 (moles acid catalyst:moles phenoxyphenone).

The process of Step (*a*) can be conducted neat although a slight amount to an excess of inert solvent

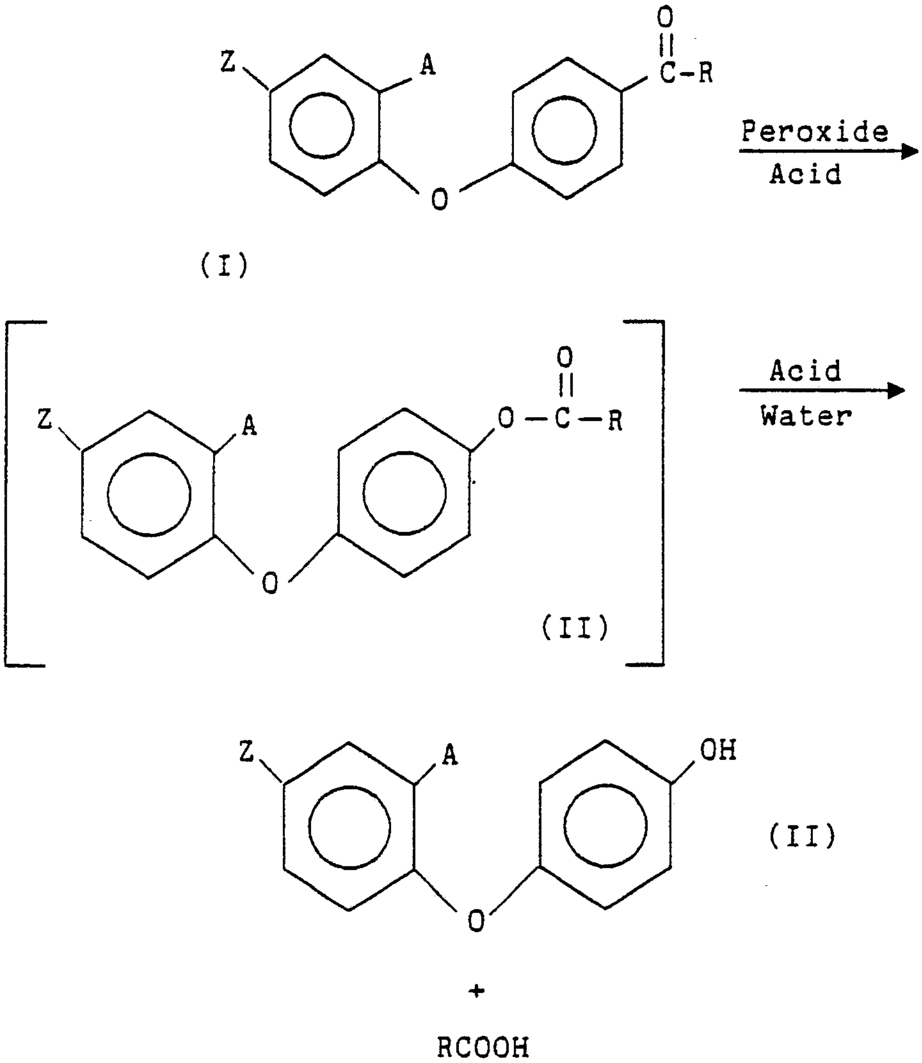


(I)

(II)

(II)

+

RCOOH

wherein Z, A and R are as defined hereinbefore.

The hydrolysis of the phenoxyphenyl ester (II) to the desired phenoxyphenol (III) occurs *in-situ* during Step (*a*) when sufficient acid is present, so that an additional separate contacting step such as in Alternatives 2 and 3 is not essential in order to prepare the desired phenoxyphenol (III). Generally, such amounts can range from stoichiometric to excess acid, preferably a slight excess. Acids which can be employed for hydrolysis include mineral acids such as sulfuric acid ($H_2SO_4$), hydrochloric (HCl), phosphoric ($H_3PO_4$) or organic acids such as acetic acid, toluene sulfonic acid, acidic ion exchange resins, such as Dowex® MSC-1-H.

During Step (*a*) wherein for the phenoxyphenone (I) R is phenyl or

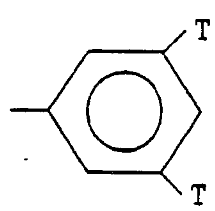

wherein T is defined as before, higher temperatures may be required than is needed for the hydrolysis where R is H or alkyl. Excess or residual peroxide need not be removed prior to the hydrolysis, although preferably, any residual or excess peroxide present with the phenoxyphenone (I) and/or phenoxyphenyl ester (II) is neutralized prior to hydrolysis to the phenoxyphenol (III). Any residual peroxide can be neutralized by adding a stoichiometric amount of a reducing agent such as sodium sulfite or sulfur dioxide.

Following neutralization of the peroxide, the phenoxyphenyl ester (II) wherein R is phenyl or

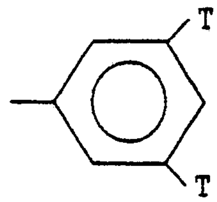

wherein T is defined as before,
is hydrolyzed *in-situ* by elevating the temperature of the reaction medium to a temperature effective to hydrolyze the phenoxyphenyl ester (III), generally between 100° to 200°C.

Recovery of the desired phenoxyphenol (III) prepared from the phenoxyphenone (I) and/or phenoxyphenyl ester (II) wherein R is H or alkyl can be accomplished by extraction procedures. After reaction, water and optionally, a water-immiscible organic solvent is added to the reaction medium, followed by separation of the organic phase containing the desired phenoxyphenol (III) and distillation thereof. Alternatively, the reaction medium can be diluted with water followed by crystallization and filtration to recover the desired phenoxyphenol (III).

Recovery of the desired phenoxyphenol (III) prepared from the phenoxyphenone (I) and/or phenoxyphenyl ester (II) wherein R is phenyl or

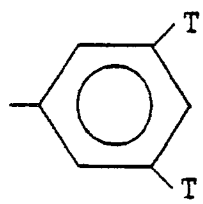

wherein T is defined as before,
can be accomplished by dilution of the reaction medium with water and separation of the organic phase. The organic phase is washed with a stoichiometric amount of a base in quantities sufficient to convert the residual organic acids such as benzoic acids to their corresponding salts and yet maintain the desired phenoxyphenol (III) in its protonated form. For example, the pH can be adjusted to 6-7 and the desired phenoxyphenol (III) can then be recovered by phase separation.

Alternative 2 - Base Hydrolysis

In alternative method 2, the phenoxyphenyl ester (II) is contacted with an aqueous base to give the phenoxyphenate (III′), followed by subsequent contacting with a suitable acid in amounts effective to yield the desired phenoxyphenol (III) as follows:

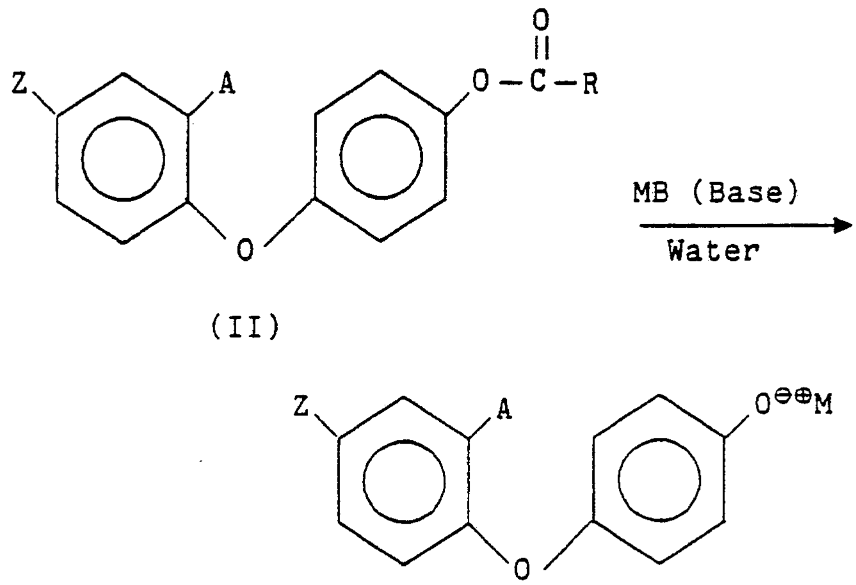


(II)

(III')

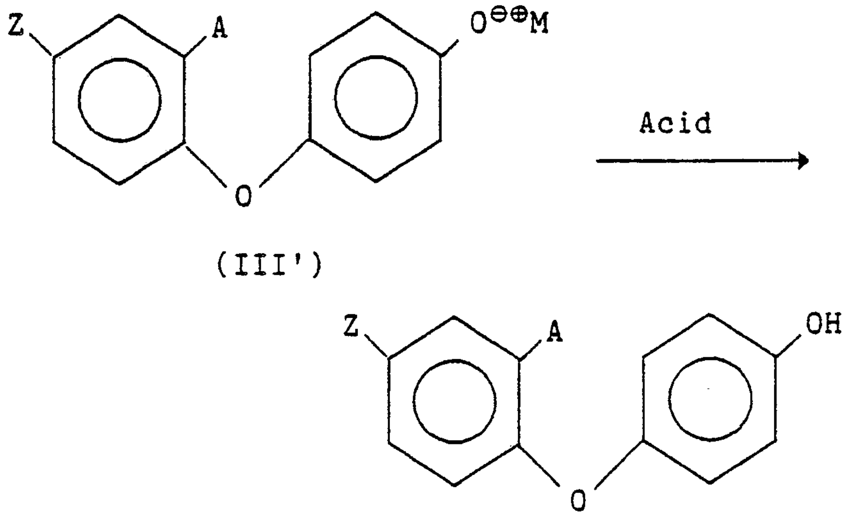


(III')

(III)

wherein Z, A and R are as defined hereinbefore.

The bases (MB) employed for converting the phenoxyphenyl ester (II) to the phenoxyphenate salt (III′) include the carbonate or hydroxide bases of the alkali and alkaline earth metals such as sodium, potassium or lithium; preferably, the hydroxide bases are employed. The base can also be anhydrous sodium or potassium methoxide. A molar excess of base relative to the phenoxyphenyl ester (II) is used to cleave the phenoxyphenyl ester (II) to the phenoxyphenolate (III′). Preferably two or more moles of base are contacted with the phenoxyphenyl ester (II).

Inert solvents can be employed in amounts sufficient to dissolve the phenoxyphenyl ester (II), the base or both. Suitable solvents include non-acidic solvents such as $C_1$-$C_6$ alcohols including methanol, ethanol, propanol, hexanol and the like; aromatic hydrocarbons such as toluene and chlorobenzene; chlorinated hydrocarbons such as methylene chloride, carbon tetrachloride, perchloroethylene and the like; and polar solvents such as dimethylsulfoxide (DMSO), tetrahydrofuran and sulfolane.

The temperatures, pressures and times employed should be sufficient to yield the desired phenoxyphenolate (III′). Such temperatures can range from 0° to 100° C, preferably from ambient to the melting point of the phenoxyphenyl ester (II). Ambient pressures are preferred although pressures greater than

ambient can be employed. The times for contacting of the ingredients can vary from about one hour to 24 hours or more.

The phenoxyphenolate (III′) can be converted to the desired phenoxyphenol (III) by contacting the phenoxyphenolate (III′) with sufficient acid to bring the pH of the reaction medium to a pH less than or equal to about 6. Generally, such amounts can range from stoichiometric to excess acid, preferably a slight excess. Acids which can be employed for converting the phenoxyphenolate (III′) to the desired phenoxyphenol (III) include mineral acids such as $H_2SO_4$, HCl, $H_3PO_4$, or organic acids such as acetic acid, toluenesulfonic acid or acidic ion exchange resins such as those described hereinbefore. The desired phenoxyphenol can be recovered by extraction, filtration and/or phase separation methods described hereinbefore.

<u>Alternative 3 - Alcoholysis (Transesterification)</u>

In this most preferred alternative method, the phenoxyphenyl ester (II) is contacted with an alcohol (R′OH) in the presence of a transesterification catalyst to yield the desired phenoxyphenol (III) as follows:

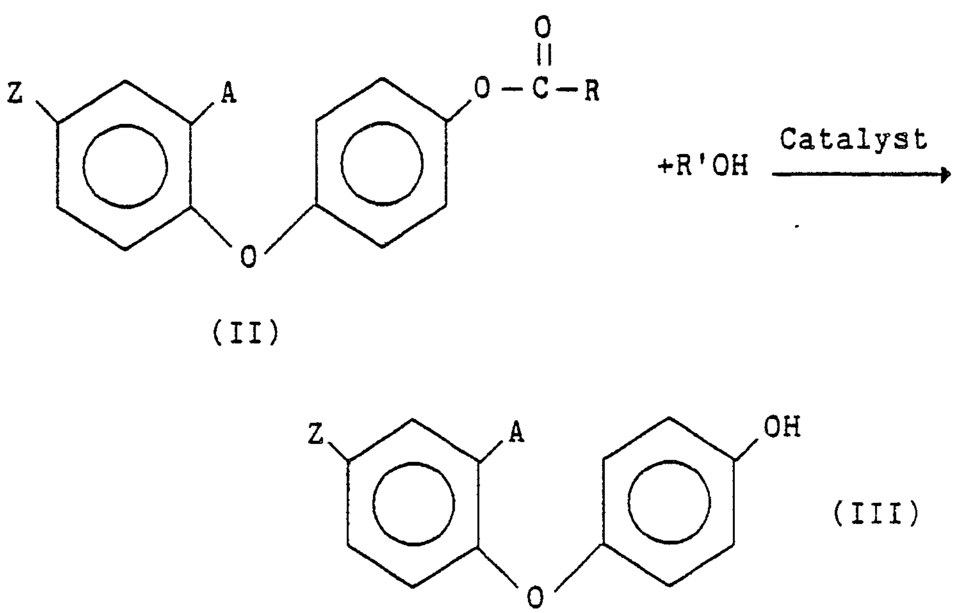


(II)

(III)

+

R'OOCR

wherein:

Z, A, and R are as defined hereinbefore; and

R′OH represents $C_1$-$C_{20}$ primary, secondary and tertiary alcohols, preferably primary alcohols, more preferably $C_1$-$C_6$ primary alcohols, most preferably hexanol.

The alcohol (R′OH) is employed in amounts sufficient to convert the phenoxyphenyl ester (II) to the desired phenoxyphenol (III). Such amounts can range from an excess of alcohol to stoichiometric amounts relative to the phenoxyphenyl ester (II) such as 100-1:1 (moles alcohol:moles phenoxyphenyl ester (II)), preferably about 5-3:1.

Catalysts which can be used to transesterify the phenoxyphenyl ester (II) to the desired phenoxyphenol (III) include mineral acids such as $H_2SO_4$, HCl and $H_3PO_4$; strong organic acids such as toluenesulfonic acid and acidic ion exchange resins described hereinbefore; and preferably tetraalkyltitanate catalysts of the formula:

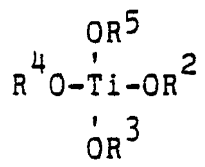

wherein $R^2$, $R^3$, $R^4$ and $R^5$ independently represent $C_1$-$C_6$ alkyl, preferably C-4; most preferably where $R^2$, $R^3$, $R^4$ and $R^5$ each is C-4 alkyl. The catalyst is used in amounts sufficient to convert the phenoxyphenyl ester (II) to the desired phenoxyphenol (III). The amounts of catalyst can range from 0.1 to 10.0 percent (weight basis) based on the weight of the phenoxyphenyl ester (II); preferably from 0.1 to 2.0 percent; more preferably from 0.1 to 0.5 percent by weight.

The temperatures, pressures and times for contacting the ingredients should be sufficient to convert the phenoxyphenyl ester (II) to the desired phenoxyphenol (III). The temperatures can range from 80 to 150 °C. Preferably the pressure employed is ambient. However, higher pressures can be employed where alcohols and low boiling point solvents are used, such as for methanol, ethanol, etc. The ingredients are contacted by mixing them for a period ranging from 0.5 hours to 24 hours or more. The transesterification can occur *in-situ* where the oxidation of step (*a*) is carried out in the presence of an alcoholic solvent (R′OH).

The desired phenoxyphenol (III) can be recovered by distillation of excess alcohol and by-products such as alkylbenzoates formed during the reaction. Crystallization and/or filtration procedures can also be employed for recovery.

The phenoxyphenol (III) may optionally be converted to the corresponding racemic or optically active (phenoxyphenoxy)propionate by conventional techniques as follows:

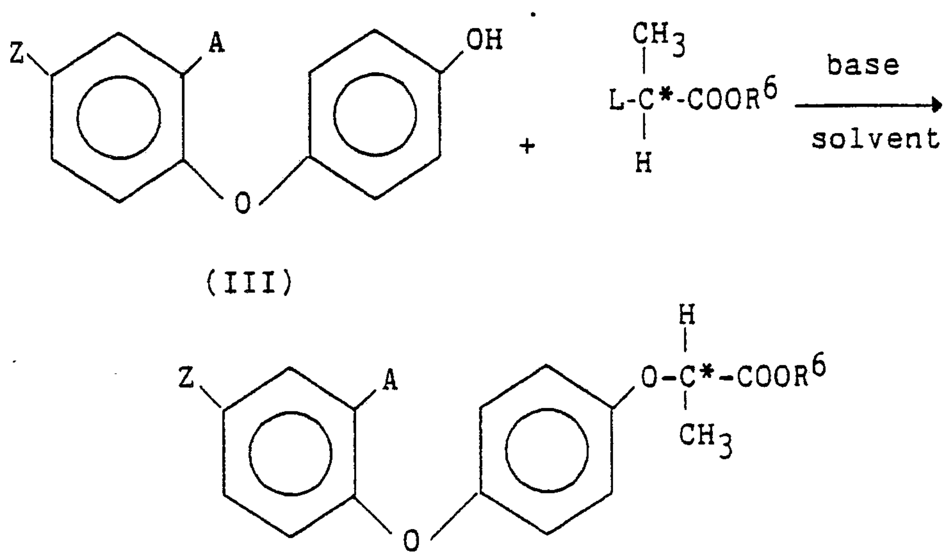


(III)

wherein:

Z and A are as described hereinbefore;

$R^6$ is hydrogen or $C_1$-$C_{10}$ alkyl;

L is halogen or an alkyl or aryl sulfonate of the formula

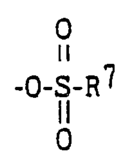

wherein:

$R^7$ is $C_1$-$C_3$ alkyl, or phenyl optionally substituted with halogen, $C_1$-$C_3$ alkyl or nitro; and

* represents an asymmetric carbon atom.

The Examples which follow illustrate the present invention, and the processes by which it can be practiced, but should not be construed as limitations upon the overall scope of the same.

Example 1 - Preparation of 4-(2-fluorophenoxy)benzophenone by coupling 2-fluorophenol and 4-chlorobenzophenone

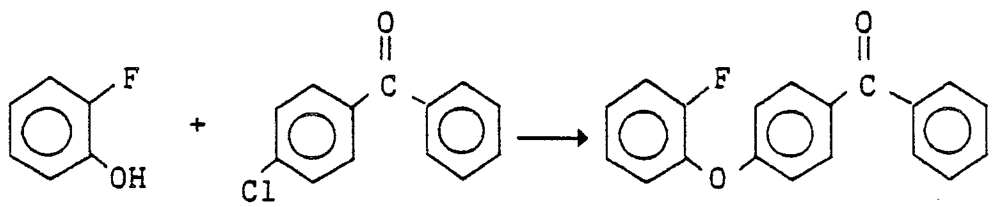

A solution of 224 grams (g) (2.00 moles) of 2-fluorophenol, 263 g of 43 percent by weight aqueous KOH (2.02 moles KOH), and one liter (1) of dimethyl sulfoxide (DMSO) were heated to 125 degrees Centigrade (°C) and water was distilled out with the aid of a Vigreux column under reduced pressure. To the reaction mixture 433 g (2.00 moles) 4-chlorobenzophenone was then added and the resulting mixture was heated for 20 hr at 125°C. The reaction mixture was diluted with 50 milliliters (ml) of concentrated hydrochloric acid (HCl) and 1 liter of water, and then extracted with two 250 ml portions of perchloroethylene. The combined organic phases were washed with 1 liter of water and then concentrated *in vacuo* to afford 581.2 g of crude product. Purification by distilling out the low boiling impurities was carried out using a column packed with perforated nickel packing. Bottoms product yield was 77 percent for 452.0 g 4-(2-fluorophenoxy)-benzophenone recovered, based on fluorophenol.

An analytical sample has a melting point of 50-52°; $^{13}$C-NMR (75.5 MHz, $CDCl_3$) $\delta$ 161.23 (s), 154.54 (d, $J_{C-F}$-250 Hz), 142.10 (d, J=12 Hz), 137.84 (s), 132.40 (s), 132.24 (s), 132.09 (s), 132.03 (s), 129.74 (s), 128.20 (s), 126.10 (d, J=8 Hz), 124.96 (d, J=4 Hz), 123.03 (s), 117.31 (d, J=18 Hz), 115.82 (s); ir (neat) 1665 $cm^{-1}$(s), 1605 (s), 1510 (s), 1455 (m), 1420 (m), 1280 (s), 1230 (s).

Example 2 - Preparation of 4-(4-bromo-2-fluorophenoxy)-acetophenone by coupling 4-bromo-2-fluorophenol with 4-fluoroacetophenone

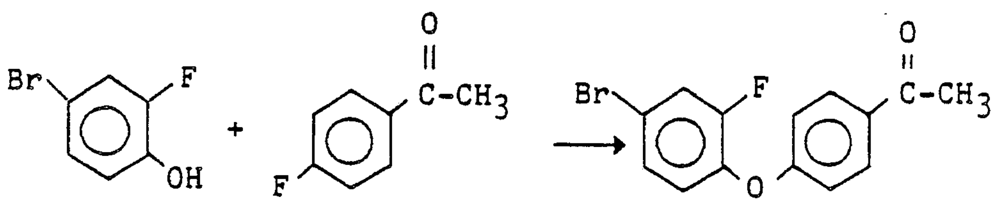

The procedure of Example 1 was employed with the following changes. Bromofluorophenol (48 g), toluene (60 ml), N-methylpyrrolidone (NMP) (50 ml), and 50 percent aqueous NaOH (17.5 g) were dried by distillation of water and toluene. Fluoroacetophenone (38.5 g) was added and the mixture was heated at 190°C for 4.5 hr. Most of the NMP was distilled out and the residue was extracted with 100 ml of cold water and 80 ml of carbon tetrachloride ($CCl_4$). The organic phase was washed with 100 ml of water, and then concentrated *in vacuo* to afford 88.2 g of crude product. Vacuum distillation afforded 52 g (76% yield) of 4-(4-bromo-2-fluorophenoxy)acetophenone, having a boiling point of 161-162°C at 0.4 millimeters (mm) Mercury (Hg).

An analytical sample has a melting point of 51-52°, IR (KBr) 1690 $cm^{-1}$ (s), 1605 (s), 1510 (s), 1420 (m), 1380 (s), 1290 (s), 1240 (s).

| | | | | |
|---|---|---|---|---|
| Elemental analysis, | | | | |
| calculated for $C_{14}H_{10}BrFO_2$: | C, | 54.37: | H, | 3.26. |
| Found: | C, | 54.42; | H, | 3.27. |

Example 3 - Preparation of 4-(2-fluorophenoxy)benzophenone by coupling 2-fluorophenol and 4-fluorobenzophenone

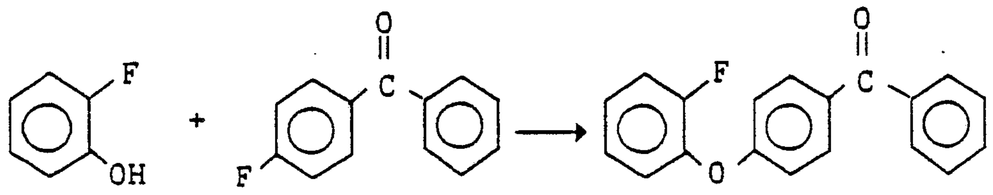

The procedure of Example 1 was employed with the following modifications.

Potassium hydroxide (12.82 g of 86.2 percent KOH; 0.197 mols) was dissolved in 11 ml of $H_2O$ and added to a solution of 40 g (0.20 moles) 4-fluorobenzophenone in 100 ml of DMSO. The mixture was degassed by three times pulling a vacuum and releasing it with $N_2$. To this solution was added, slowly, 21.5 g (0.19 moles) of *o*-fluorophenol and 2.1 g (0.0095 moles) BHT (2,6-di-*t*-butyl-4-methylphenol). The bulk of the water was removed by vacuum distillation, keeping the pot temperature under 85 °C. The water distillation took 2 hr and 12.2 g of distillate was collected. The reaction mixture, which analyzed at 0.8% $H_2O$, was held at 80 °C for 19 additional hr, at which time conversion was approximately 99 percent. The reaction mixture was diluted with 60 ml $H_2O$, 4 ml conc. HCl, and 20 ml perchloroethylene. The aqueous phase was extracted with an additional 10 ml of perchloroethylene and the combined organic phases were washed with 30 ml $H_2O$. Solvent stripping left 59.5 g of oil, which solidified upon standing. The crude product assayed as 92.0 percent pure 4-(2-fluorophenoxy)benzophenone, for a 98 percent isolated yield.

Example 4 - Preparation of 4-(2-fluorophenoxy)benzophenone by coupling 2-fluorophenol and 4-fluorobenzophenone

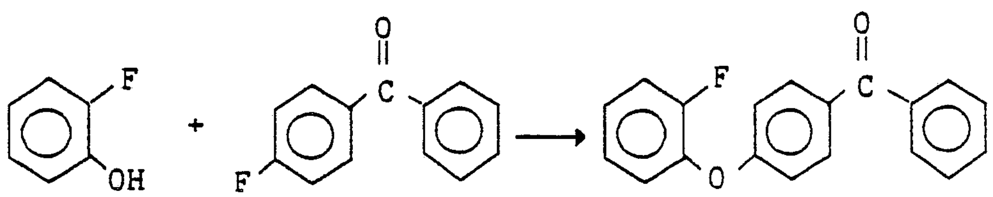

The procedure of Example 1 was employed with the following modifications.

All the components (301 g of 46 percent aqueous KOH, 501 g of fluorobenzophenone, 800 ml of DMSO, and 269 g of fluorophenol), plus 5 mole percent BHT (25 g) were mixed together under a nitrogen atmosphere and distillation of water was carried out at a maximum temperature of 80 °C. Reaction continues at 70 °C for a total of 12 hr. Purification by lights distillation gave an 87 percent yield of 4-(2-fluoro-phenoxy)benzophenone.

Example 5 - Preparation of 4-(4-bromo-2-fluorophenoxy)benzaldehyde by coupling 4-bromo-2-fluorophenol and 4-fluorobenzaldehyde

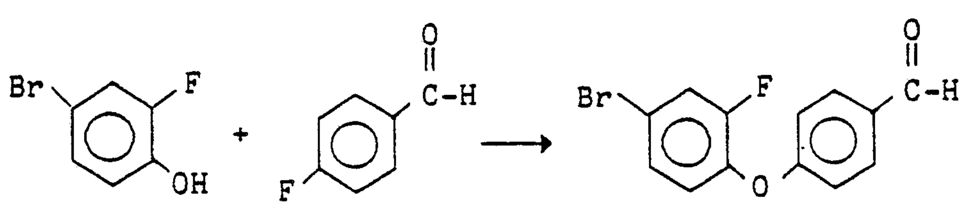

Sodium 4-bromo-2-fluorophenate was prepared by contacting 25 percent aqueous NaOH and bromofluorophenol (1.11 equivalents). The resulting solution was washed with toluene and the aqueous product layer was concentrated *in vacuo*. The solid product was powdered and contained 2.4 percent water. A solution of 10.0 g of the sodium bromofluorophenate in 20 ml of NMP was heated to 120° C and 5.8 g of fluorobenzaldehyde was added. After the mixture was heated at 180° C for 4 hr, it was diluted with 100 ml of 10 percent HCl and extracted with $CH_2Cl_2$. The organic layer was washed with 100 ml of water and then concentrated *in vacuo*. Vacuum distillation of the crude product affords 10.4 g of 4-(4-bromo-2-fluorophenoxy)benzaldehyde, bp 145-152° /0.6 mm Hg.

Example 6 - Preparation of 4-(4-bromo-2-fluorophenoxy) benzophenone via bromination of 4-(2-fluorophenoxy) benzophenone

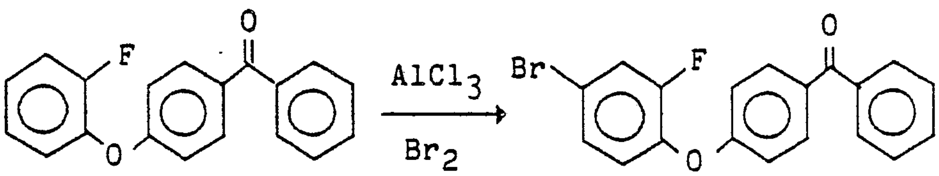


To a slurry of 295 g of $AlCl_3$ powder (2.22 moles) in 1.5 liter of methylene chloride ($CH_2Cl_2$) was added 581 g of fluorophenoxybenzophenone (2.00 moles). After the mixture becomes homogeneous, a solution of 329 g of bromine (2.06 moles) in 300 ml of $CH_2Cl_2$ was added continuously over a period of 3 hr while maintaining a reaction temperature of 30° C. The reaction mixture was continuously added to 2.2 kg of ice water. The organic phase was separated and washed with 1 liter of water.

The resulting solution of 4-(4-bromo-2-fluorophenoxy)benzophenone (~-95% purity by capillary gas chromatographic analysis) in $CH_2Cl_2$ was carried forward to the next step (Example 9). A purified sample has a melting point of 91.0-92.5° C; IR (KBr) 1650 (s), 1610 (s), 1590 (s), 1500 (s), 1290 (s), 1275 (s), 1220 (s).

| | | | | |
|---|---|---|---|---|
| Elemental analysis, calculated for $C_{19}H_{12}BrFO_2$: | C, | 61.46; | H, | 3.26. |
| Found: | C, | 61.61; | H, | 3.32. |

Example 7 - Preparation of 2-fluorodiphenyl oxide

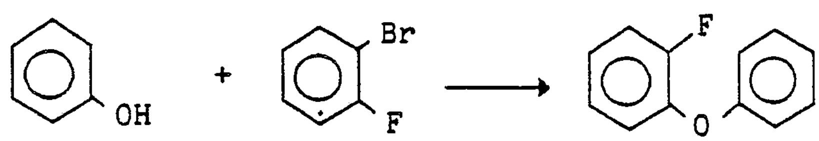

A mixture of phenol (47.5 g; 0.5 moles), butylated hydroxytoluene (BHT; 2.0 g; 0.009 moles), diglyme (200 ml) and 25 percent $NaOCH_3/CH_3OH$ (111.8 g; 0.5 moles) in a nitrogen purged flask was heated to 120° C to remove the methanol (104.4 g distillate). After cooling to 75° C, *o*-bromofluorobenzene (134 g; 0.77 moles) copper metal (0.5 g; 0.008 moles) and cuprous chloride (1.9 g; 0.019 moles) were added and the mixture was heated at 125° C. After 20 hr, gas chromatographic analysis indicated 50 percent conversion and the reaction mixture was quenched with 40 ml of water and 10 ml of concentrated hydrochloric acid. After filtration, the organic phase was separated and distilled to give three cuts: 60g of diglyme and o-bromofluorobenzene; 27.8 g of primarily phenol; and 49.5 g of 98 percent pure 2-fluorodiphenyl oxide.

Example 8 - Preparation of 4-(4-bromo-2-fluorophenoxy)-3′,5′dichlorobenzophenone

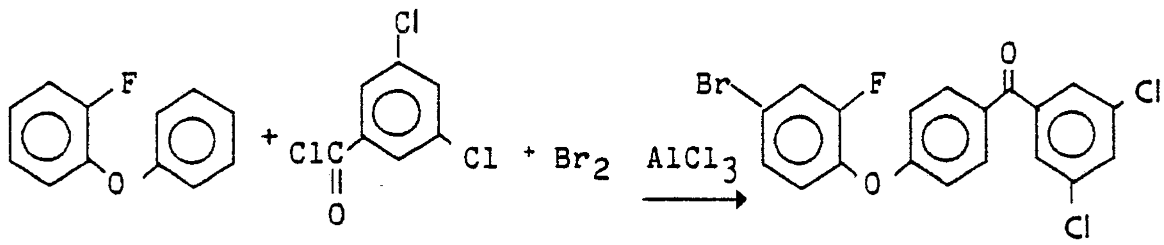


To a mixture of 1.30 g of 2-fluorodiphenyl oxide, 1.49 g of 3,5-dichlorobenzoyl chloride, and 4 ml of carbon tetrachloride was added 1.0 g of aluminum chloride in portions over 20 min with ice-water cooling. After two hr, a solution of 1.2 g of bromine in 1 ml of carbon tetrachloride was added over 30 min with ice-water cooling. After an additional 3.5 hr, the reaction mixture was poured into water and extracted with carbon tetrachloride. The combined organic phase was washed with dilute sodium thiosulfate solution and concentrated *in vacuo*, yielding 3.0 g. A portion of this product was recrystallized from carbon tetrachloride (mp 126 .5-128.5 °C).

Example 9 - Oxidation of 4-(4-bromo-2-fluorophenoxy)-3′,5′-dichlorobenzophenone

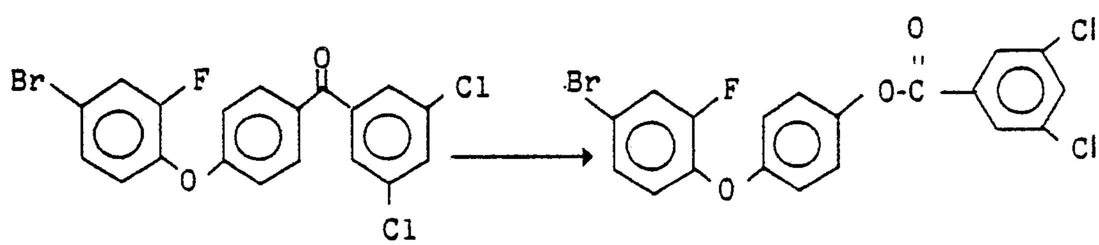

To a slurry of 1.0 g of 4-(4-bromo-2-fluorophenoxy)-3′,5′-dichlorobenzophenone in 6 ml of acetic acid was added 1.6 g of sulfuric acid and the mixture was heated to 50 °C. Hydrogen peroxide (70%, 0.20 g) was added over 0.5 hr. Heating continued for 7 hr and then the reaction mixture was allowed to stir for 2.5 days at ambient temperature. At this time, the reaction mixture was partitioned between methylene chloride and water, and the organic phase was concentrated *in vacuo* to give 0.93 g of 4-(4-bromo-2-fluorophenoxy)phenyl-3′,5′-dichlorobenzoate as a yellow-white solid which was 96 area % pure by capillary GC analysis. The structure of this product was confirmed by performing a basic hydrolysis (25% aq KOH, ethanol, 70 °C, 3 hr) and obtaining 4-(4-bromo-2-fluorophenoxy)-phenol along with 3,4-dichlorobenzoic acid.

Example 10 - Preparation of 4-(4-bromo-2-fluorophenoxy)phenyl benzoate with peracetic acid generated *in situ* from hydrogen peroxide and acetic acid

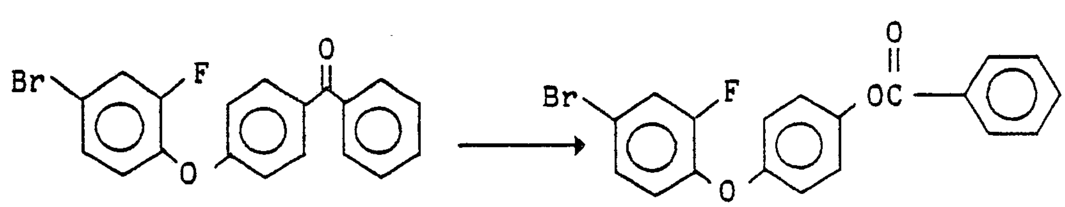

Most of the $CH_2Cl_2$ is stripped from one-half of the solution containing 4-(4-bromo-2-fluorophenoxy)-benzophenone from Example 5. To the resulting crude 4-(4-bromo-2-fluorophenoxy)benzophenone (370 g)

was added 1086 ml of glacial acetic acid and then 186 ml of concentrated sulfuric acid. The reaction mixture was heated at 50° C and 58 g of 70 percent aqueous hydrogen peroxide was added over a period of 2.5 hr. After 7 hr, one liter of water was added and the mixture was then extracted with 200 ml of perchloroethylene. The organic phase was then washed successively with 500 ml of 10 percent aqueous sodium carbonate and 500 ml of 1 percent aqueous sodium bisulfite. After removal of solvent, the crude 4-(4-bromo-2-fluorophenoxy)phenyl benzoate weighed 360.1 g (90% purity, 88% yield). A purified sample has a melting point of 72-75° C, ir (KBr) 1745 $cm^{-1}$ (s), 1600 (m), 1505 (s), 1390 (s), 1205 (s), 1100 (s), 1080 (s).

Example 11 - Preparation of 4-(4-bromo-2-fluorophenoxy)phenol from 4-(4-bromo-2-fluorophenoxy)-acetophenone and peracetic acid

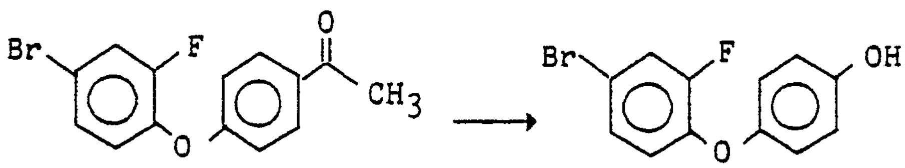

To a solution of 6.3 g of 4-(4-bromo-2-fluorophenoxy)acetophenone, 20 g of acetic acid, and 1.1 g of concentrated sulfuric acid was added 1.4 g of 70 percent hydrogen peroxide over a period of 20 min. After one day (about 24 hr) at ambient temperature, the reaction mixture was poured into 150 ml of ice-water, and the solid which formed was collected by suction filtration. After air drying 5.4 g of crude 4-(4-bromo-2-fluorophenoxy)phenol of 86% purity (82% yield) was obtained.

Example 12 - Preparation of 4-(4-bromo-2-fluorophenoxy)phenol by base hydrolysis of 4-(4-bromo-2-fluorophenoxy)phenyl benzoate

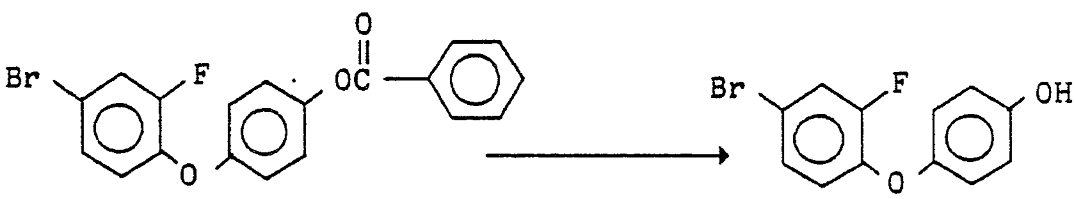

The crude bromofluorophenoxyphenyl benzoate (99 g) from an oxidation reaction similar to Example 9 was slurried in 500 ml of methanol and 350 g of 11 percent aqueous KOH was added while maintaining a temperature of 18-27° C. After 11 hr, the reaction mixture was diluted with one liter of water and the pH was adjusted to 7 with concentrated HCl. Methylene chloride (20 ml) was added, the phases separated, and the solvent removed *in vacuo* to give 68.3 g of 4-(4-bromo-2-fluorophenoxy)phenol (95% purity, 94% yield). A purified sample has a melting point of 83.5-85.0° C; $^{13}$C-NMR (20.15 MHz, $CDCl_3$) $\delta$ 153.6 (d, $J_{C-F}=253$ Hz), 151.7 (s), 150.1 (s), 144.6 (d, J=11 Hz), 127.6 (d, J=4 Hz), 121.2 (s), 120.4 (d, J=21 Hz), 119.6 (s), 116.5 (s), 115.1 (d, J=8 Hz).

Example 13 - Preparation of 4-(4-bromo-2-fluorophenoxy)phenol by alcoholysis (transesterification) of 4-(4-bromo-2-fluorophenoxy)phenyl benzoate with hexanol and tetrabutyltitanate

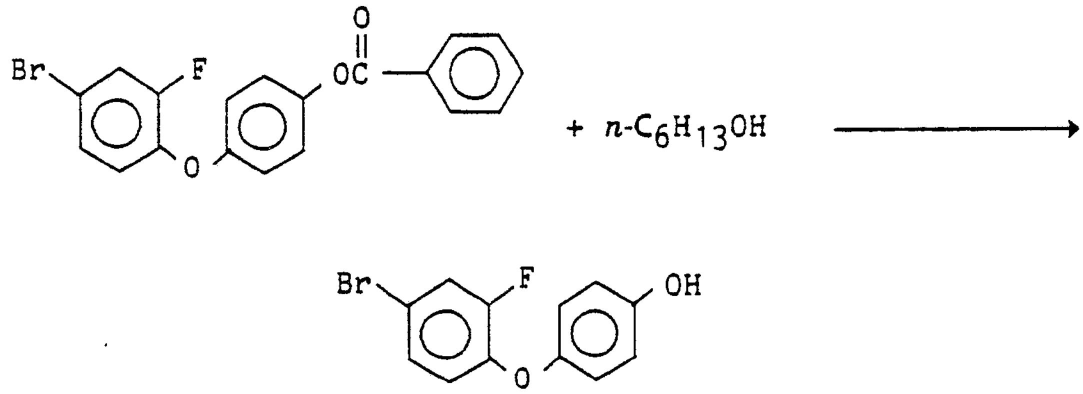


A solution of crude 4-(4-bromo-2-fluorophenoxy)phenyl benzoate (360 g) and hexanol (360 g) was dried by distilling out 30 ml of hexanol and then 0.8 g of tetrabutyltitanate was added and the reaction mixture was heated for 38 hr at 115°C. A 93 g sample was removed at this time. Water (0.2 g) was then added, followed by removal of unreacted hexanol and the byproduct hexyl benzoate via vacuum distillation continued to produce 183 g of 4-(4-bromo-2-fluorophenoxy)phenol of 97% purity (86% yield).

Example 14 - Preparation of 4-(4-bromo-2-fluorophenoxy)phenol from 4-(4-bromo-2-fluorophenoxy)-acetophenone and hydrogen peroxide

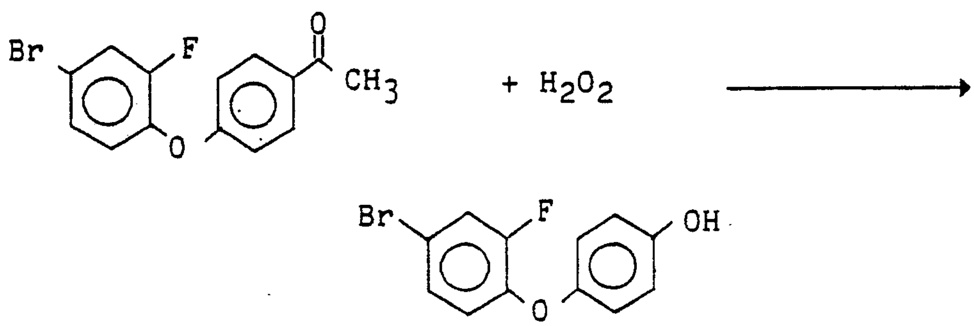


To a solution of 0.70 g of bromofluoroacetophenone, 4 ml of methanol, and 0.60 g of concentrated sulfuric acid was added 0.30 g of 70 percent hydrogen peroxide. This solution was heated for 3 hr at 50°C and then diluted with 5 ml of $CH_2Cl_2$ and washed successively with dilute aqueous $Na_2SO_3$ (10 ml) and water (10 ml). Solvent was removed *in vacuo* to give 0.58 g of 4-(4-bromo-2-fluorophenoxy)phenol of 62 percent purity by capillary gas chromatographic analysis.

## Claims

1. A phenoxyphenone compound of the formula:

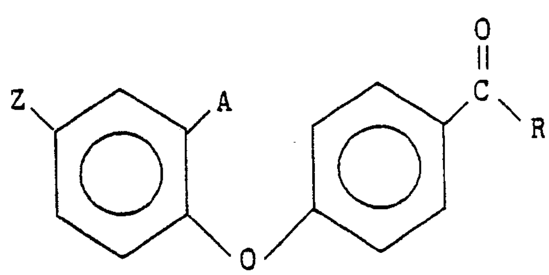 (I)

wherein:
Z is halogen or H;
A is halogen or H; and
R is H, $C_1$-$C_{10}$ alkyl, phenyl or substituted phenyl of the formula

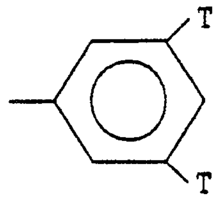

wherein T is independently halogen or H.

2. The compound of Claim 1 wherein 2 is halogen, A is halogen and R is methyl or phenyl.

3. The compound of Claim 2 wherein A is fluorine.

4. The compound of Claim 2 wherein 2 is bromine and A is fluorine.

5. A phenoxyphenyl ester compound of the formula:

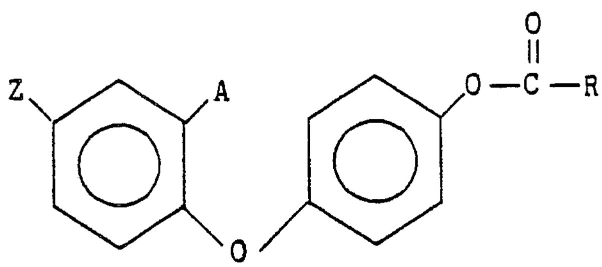

(II)

wherein:
Z is halogen or H;
A is halogen or H; and
R is H, $C_1$-$C_{10}$ alkyl, phenyl or substituted phenyl of the formula

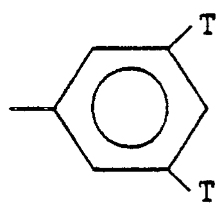

wherein T is independently halogen or H.

6. The compound of Claim 5 wherein 2 is halogen, A is halogen and R is methyl or phenyl.

7. The compound of Claim 6 wherein A is fluorine.

8. The compound of Claim 6 wherein Z is bromine and A is fluorine.

9. A compound of the formula

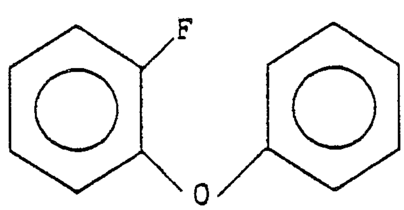

10. A process for preparing a 4-bromophenoxyphenone compound of the formula:

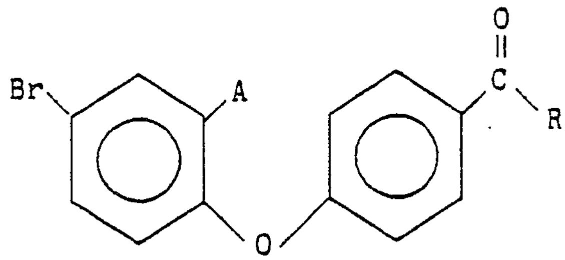

(I'')

wherein A and R are defined as in Claim 1, which comprises reacting a 4-hydrogen phenoxyphenone compound of the formula

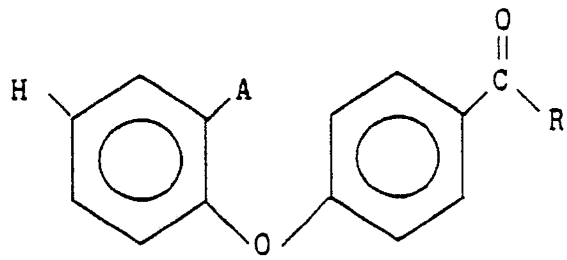

(I')

wherein:
A is halogen or hydrogen; and
R is H, $C_1$-$C_{10}$ alkyl, phenyl or substituted phenyl of the formula

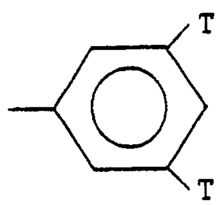

wherein T is independently halogen or H,
with bromine in the presence of a metal halide catalyst and solvent, wherein the metal halide catalyst is employed in amounts greater than the molar equivalents of 4-hydrogen phenoxyphenone (I') employed.

11. The process of Claim 10 wherein the metal halide catalyst is of the formula
$M^nX_n$
wherein:
M is aluminum, titanium iron or boron (B);
X is chloro, bromo or fluoro; and
n is an integer which is the oxidation state of the metal.

12. The process of Claim 11 wherein M is aluminum, X is chloro or bromo and n is 3.

13. The process of Claim 11 wherein A is fluoro and R is methyl or phenyl.

14. A process for preparing a phenoxyphenol compound of the formula

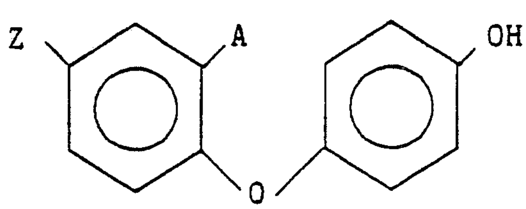

(III)

wherein:
Z is halogen or H; and

A is halogen or H,

which comprises

(a) reacting a phenoxyphenone compound of the formula

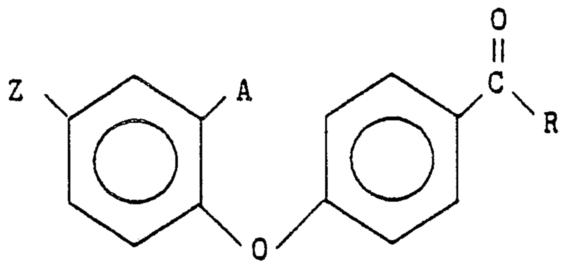

(I)

wherein:

Z and A are as defined in Claim 1, and

R is H, $C_1$-$C_{10}$ alkyl, phenyl or substituted phenyl of the formula

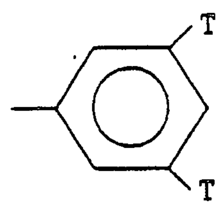

wherein T is independently H or halogen,

with peroxide in the presence of an acid catalyst to form a phenoxyphenyl ester of the formula

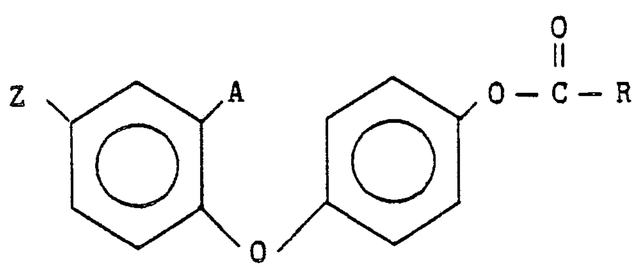

(II)

wherein:

Z, A, and R are as defined in Claim 5; and

(b) converting the phenoxyphenyl ester (II) to the phenoxyphenol (III) by conventinal means.

15. The process of Claim 14, step (b), wherein the phenoxyphenyl ester is converted to the phenoxyphenol by alcoholysis with an alcohol in the presence of a transesterification catalyst.

16. The process of Claim 13 wherein the transesterification catalyst is a tetraalkyltitanate.

17. The process of Claim 14, step (b), wherein the phenoxyphenyl ester is converted to the phenoxyphenol by hydrolysis in the presence of an acid.

18. The process of Claim 14, step (b), wherein the phenoxyphenyl ester is converted to the phenoxyphenol by hydrolysis in the presence of a base followed by neutralization with an acid.

19. The process of Claim 14 wherein Z is halogen, A is halogen and R is methyl or phenyl.

20. The process of Claim 18 or 19 wherein A is fluorine.

21. The process of Claim 20 wherein Z is bromine and A is fluorine.

European Patent Office

# EUROPEAN SEARCH REPORT

Application Number

EP 88 20 3021

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | FR-A-2 085 712 (BOOTS PURE DRUG CO)<br>* page 11, line 41 *<br>--- | 1 | C 07 C 47/575<br>C 07 C 49/84<br>C 07 C 43/29<br>C 07 C 69/78<br>C 07 C 45/63<br>C 07 C 67/42<br>C 07 C 37/055 |
| X | GB-A-2 050 168 (SHELL INTERNATIONAL RESEARCH)<br>* page 3, line 48; page 4, line 40 *<br>--- | 1 | |
| X | EP-A-0 178 184 (RAYCHEM CORP.)<br>* page 26, example 13 *<br>--- | 1 | |
| X | PATENT ABSTRACTS OF JAPAN<br>vol. 9, no. 36 (C-266)(1759), 15th February 1985; & JP - A- 59 179 570 (MOROHOSHI INK K.K.) 12.10.84<br>--- | 1 | |
| X | DE-A- 650 430 (CHEMISCHE FABRIK VON HEYDEN)<br>* claim *<br>--- | 1 | |
| X | EP-A-0 069 597 (RAYCHEM CORP.)<br>* page 2, lines 1-8; pages 6-7, example 1 *<br>--- | 1 | **TECHNICAL FIELDS SEARCHED (Int. Cl.4)** |
| X | EP-A-0 197 788 (RAYCHEM CORP.)<br>* pages 7-8, example 1 *<br>--- | 1 | C 07 C 47/00<br>C 07 C 49/00<br>C 07 C 67/00<br>C 07 C 37/00<br>C 07 C 69/00 |
| X | EP-A-0 133 244 (DU PONT)<br>* scheme 3, page 17; page 23, examples 20,23,29,35 *<br>--- | 1 | |
| X | US-A-3 939 278 (LACEFIELD)<br>* page 5, lines 25-49 *<br>--- | 1 | |
| X<br>A | EP-A-0 035 768 (HOFFMANN - LA ROCHE)<br>* page 14, lines 21-28 *<br>--- -/- | 5<br>1 | |

The present search report has been drawn up for all claims

| Place of search | Date of completion of the search | Examiner |
| --- | --- | --- |
| BERLIN | 07-03-1989 | PROBERT C.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 20 3021

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 418 480 (RICHARDSON-MERRELL)<br>* page 19, example 8 *<br>--- | 1 | |
| X | TETRAHEDRON<br>vol. 40, no. 10, 1984, pages 1919-1925, Pergamon Press, Oxford, GB; D.E. AMES et al.:" Palladium-Catalysed cyclisation of 2-substituted halogenoarenes by dehydrohalogenation".<br>* page 1919, compound 4 f *<br>--- | 1 | |
| X | CHEMICAL ABSTRACTS<br>vol. 93, no. 6, 11th August 1980, page 797, abstract no. 58557y, Columbus, Ohio, US; C. DESTRADE et al.:"Mesogenic and nonmesogenic central rigidcores" & Mol. Cryst. Liq. Cryst. 1980, 59 (3-4) 273-88 & Chemical Abstracts Formula Index 10th Collective (1981), page 13451F, column 1, lines 81-82<br>--- | 1 | |
| X | GB-A-1 543 964 (I.C.I.)<br>* page 29, compounds 121/122 *<br>--- | 1 | TECHNICAL FIELDS SEARCHED (Int. Cl.4) |
| X | DE-A-2 538 016 (HOECHST)<br>* page 5, examples 32-34 * | 5 | |
| A | --- -/- | 1 | |

The present search report has been drawn up for all claims

| Place of search | Date of completion of the search | Examiner |
|---|---|---|
| BERLIN | 07-03-1989 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 20 3021

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS vol. 68, no. 23, 3rd June 1968, page 10071, abstract no. 104463y, Columbus, Ohio, US; L.A. MATTANO et al.:"The synthesis and saponification kinetics of some 4'-substituted 4-phenolxyphenyl acetates." & Canadian Journal of Chemistry 46(7) 1156-8 (1968) & CA 8th Collective Formula Index (1971), page 4948F, column 2, lines 110-111; page 5011F, column 2, lines 81-82 * | 1 | |
| X | idem --- | 5 | |
| A | FR-A-2 242 078 (SANDOZ) * page 1, lines 28-29 * --- | 1 | |
| A | EP-A-0 189 044 (BAYER) * page 23, lines 1-11 * --- | 1 | |
| A | GB-A-1 471 012 (SANDOZ) * claim 1 * --- | 1 | TECHNICAL FIELDS SEARCHED (Int. Cl.4) |
| A | METHODEN DER ORGANISCHEN CHEMIE vol. VI,VII, 4th edition, 1976, Georg Thieme Verlag, Stuttgart, D; HOUBEN-WEYL et al.:"Methoden der organischen Chemie". * vol. VI/1C, page 436, paragraph 2; page 439, paragraph 5; vol. VII/2B, page 1985, lines 9-11 * --- | 14 | |
| A,P | EP-A-0 268 073 (ROEHM) * page 3, lines 1-17 * --- | 14 | |
| A | EP-A-0 211 561 (I.C.I.) * pages 122-123, example 3 * --- -/- | 10 | |

The present search report has been drawn up for all claims

| Place of search | Date of completion of the search | Examiner |
|---|---|---|
| BERLIN | 07-03-1989 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 88 20 3021

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 190 815 (CELANESE CORPORATION)<br>* claim 4 *<br>--- | 14 | |
| A | WO-A-8 600 301 (NESTE OY)<br>* claim 1 *<br>----- | 16 | |

TECHNICAL FIELDS SEARCHED (Int. Cl.4)

The present search report has been drawn up for all claims

| Place of search | Date of completion of the search | Examiner |
|---|---|---|
| BERLIN | 07-03-1989 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document